# EUROPÄISCHE PATENTSCHRIFT

(11) **EP 1 561 431 B1**
(45) Veröffentlichungstag und Bekanntmachung des Hinweises auf die Patenterteilung: **12.08.2009**
(21) Anmeldenummer: 04002321.0
(22) Anmeldetag: 03.02.2004
(51) Int. Cl.: A61B 19/00

(54) **Vorrichtung zum Bestimmen der Position eines Schneidblocks**
Device for determining the position of a cutting guide
Dispositif pour déterminer la position d'un guide de coupe

(43) Veröffentlichungstag der Anmeldung: 10.08.2005
(73) Patentinhaber: BrainLAB AG, 85622 Feldkirchen (DE)
(72) Erfinder: Plassky, Norman, 99099 Erfurt (DE); Neubauer, Timo, 85622 Feldkirchen (DE)
(74) Vertreter: Gassenhuber, Andreas

(56) Entgegenhaltungen:
- EP-A- 1 249 207
- WO-A-00/41635
- WO-A-20/04017842
- WO-A-20/04082489
- FR-A- 2 830 743
- US-A1- 2002 107 522
- US-B2- 6 551 325

## Beschreibung

Die vorliegende Erfindung bezieht sich auf eine Vorrichtung zum Bestimmen der Position eines medizinischen Operationsinstrumentes, wie zum Beispiel eines Schneidblockes und kann auch zum Bestimmen der Position von Oberflächen, wie zum Beispiel einer Knochenoberfläche verwendet werden. Insbesondere bezieht sich die Erfindung auf eine Vorrichtung, welche beim Ermitteln einer räumlichen Position oder bei der Positionierung eines Elements verwendet werden kann.

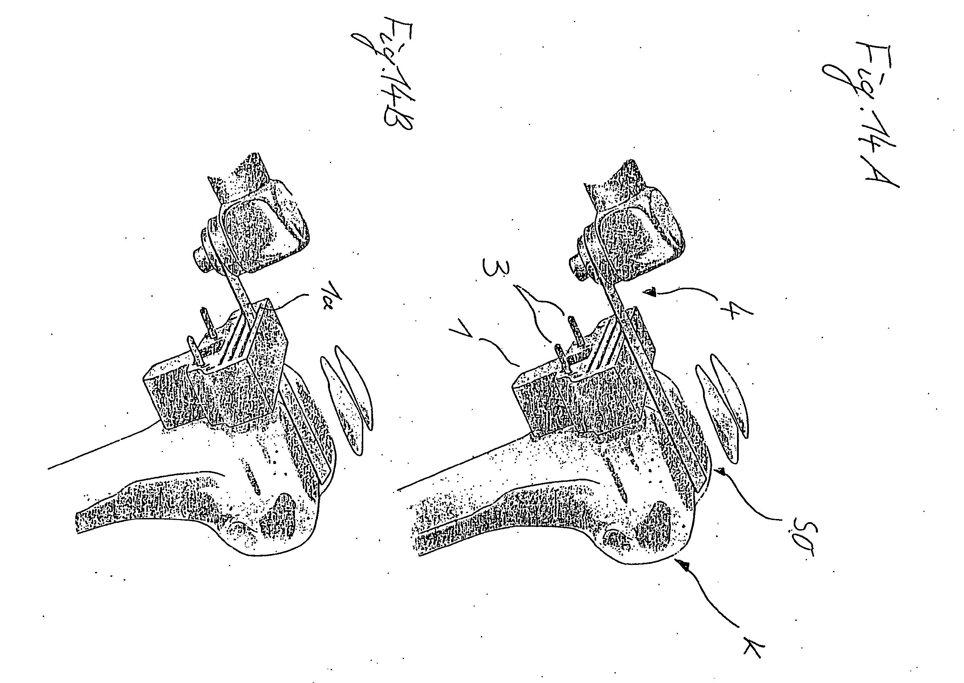

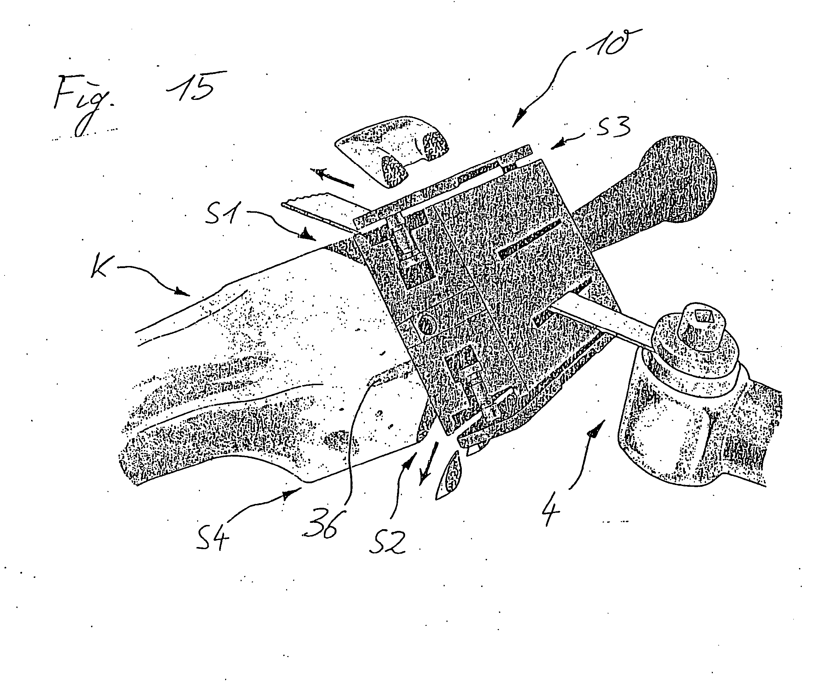

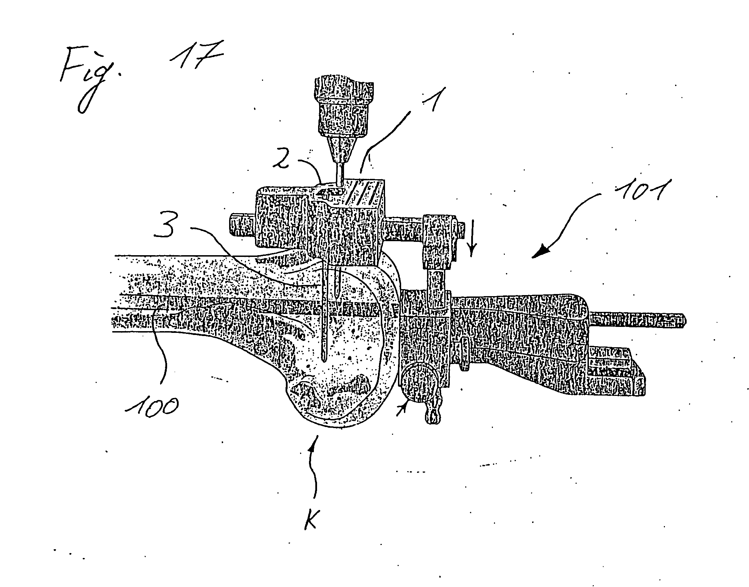

Beim Anbringen von Implantaten, wie zum Beispiel künstlichen Knie-, Ellbogen, Fingeroder Hüftgelenken, ist es erforderlich das Implantat, wie zum Beispiel ein Gelenk oder ein Knochenteil, möglichst genau am angrenzenden Knochen zu positionieren. Hierzu müssen möglichst exakte Schnitte an den an das Gelenk angrenzenden Knochenstrukturen ausgeführt werden. Figur 17 zeigt schematisch die Positionierung eines Schneidblockes 1 an einem Oberschenkelknochen K nach dem Stand der Technik. Hierzu wird eine Führungsstange 100 in einen Knochen K eingebracht, wobei am äußeren Ende der Führungsstange 100 eine Positionierungsmechanik 101 zum Positionieren und Halten des Schneidblockes 1 in einer gewünschten Position vorgesehen ist. Ist der Schneidblock 1 in die gewünschte Position gebracht worden, so können durch in dem Schneidblock 1 vorgesehene Löcher 2 Positionierungsstifte 3, beispielsweise Schrauben oder Nägel bzw. Pins in den Knochen K zur Fixierung des Schneidblocks 1 eingebracht werden. Nach erfolgter Fixierung des Schneidblockes 1 am Knochen K kann eine bevorzugt möglichst senkrecht oder leicht zur mechanischen Achse des Knochens abgewinkelt liegende erste Schnittebene S0 mittels eines Schneidwerkzeuges 4 erzeugt werden, wie schematisch in den Figuren 14A und 14B dargestellt. Um im Beispiel des Oberschenkelknochens die dazugehörige Knieimplantatkomponente auf dem Knochen positionieren zu können, müssen weitere Schnitte in weiteren zur Schnittebene S0 schräg verlaufenden oder abgewinkelten Ebenen S1 bis S4 erzeugt werden, wie in Figur 15 gezeigt. Hierzu wird ein zweiter Schneidblock 10 auf die erste Schnittebene S0 aufgesetzt und mittels einer geeigneten Mechanik positioniert, wobei mit diesem zweiten Schneidblock 10 weitere seitliche Schnitte am Knochen K erzeugt werden, welche durch Führung des Schneidwerkzeuges 4 in den schräg verlaufenden oder seitlichen Schlitzen des zweiten Schneidblockes 10 erzeugt werden.

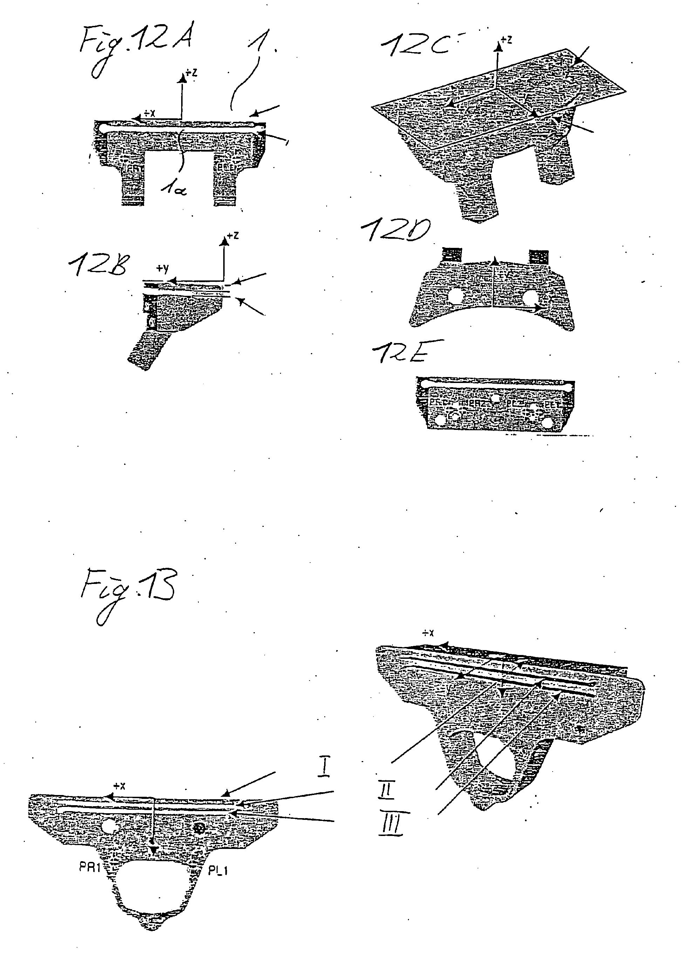

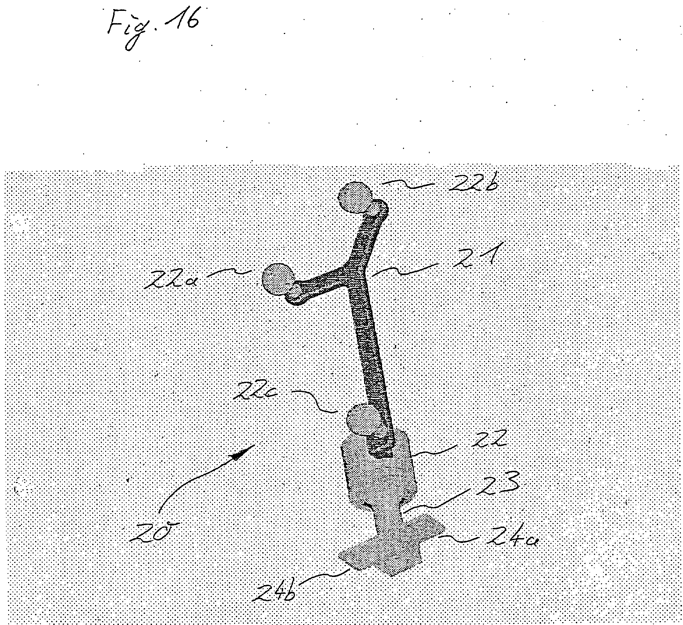

Aus der EP 1 190 676 A1 und der korrespondierenden US 6,551,325 der Anmelderin ist eine Vorrichtung zum Bestimmen der Position eines Schneidblockes wie in Figur 16 gezeigt bekannt, wobei die Vorrichtung einen Referenzstern 21 und darauf angeordnete kugelförmige Elemente 22a bis 22c mit reflektierender Oberfläche aufweist, wobei zwei nicht gezeigte Infrarotkameras ein an den kugelförmigen Elementen 22a bis 22c reflektiertes Licht erfassen und hieraus die Lage des Referenzsternes 21 im Raum ermitteln. Der Referenzstern 21 ist fest mit dem Grundkörper 22 verbunden, an welchem ein drehbares Element 23 angebracht ist, wobei am äußeren Ende des drehbaren Elementes 23 zwei Platten 24a und 24b unterschiedlicher Dicke angeordnet sind, welche in einen Führungsschlitz 1a des in den Figuren 12A bis 12E gezeigten tibialen Schneidblockes oder einen der Führungsschlitze II, III des in Figur 13 gezeigten femoralen Schneidblockes eingesteckt werden können. Zum Erzeugen des in Figur 14 gezeigten Schnittes muss der mit der eingesteckten Vorrichtung 20 verbundene Schneidblock an die gewünschte Stelle des Knochens K navigiert werden, wo der Schneidblock 1 mit Befestigungselementen 3 am Knochen K befestigt werden kann, wie in Figur 14A gezeigt. Mit einem Schneidwerkzeug 4 kann anschließend ein Schnitt in der gewünschten Schnittebene, wie in Figur 14B gezeigt, erzeugt werden. Auf der so erzeugten Schnittebene des Knochens A kann ein zweiter Schneidblock 10, wie schematisch in Figur 15 gezeigt, aufgesteckt werden und durch in dem zweiten Schneidblock 10 vorgesehene Führungsschlitze können Schnitte in den Ebenen S1 bis S4 ausgeführt werden, so dass zum Beispiel ein künstliches Gelenk aufgesetzt werden kann, welches bei richtiger Lage der Schnittebene S0 bis S4 auch richtig positioniert ist.

Es kann jedoch bei mehrfacher Verwendung der oben beschriebenen Positionierungsvorrichtung 20 zu Abnutzungen im Bereich der in Figur 16 gezeigten Platten 24a und 24b oder zu einer Vergrößerung der Führungsschlitze 1a durch das Schneidwerkzeug 4 kommen, was dazu führen kann, dass diese Platten 24 nicht mehr formgenau in einen Schlitz eines Schneidblockes eingesteckt werden können, so dass der Schneidblock zum Beispiel aufgrund eines vorhandenen Spiels und damit eines lockeren Sitzes ungenau positioniert wird.

Aus der FR 2830743 A1 ist ein Tastinstrument mit drei Markern bekannt, wobei eine Kontaktkugel des Tastinstruments mittels einer Druckfeder aus einem Gehäuse des Instruments herausgedrückt und einer der Marker durch die Druckfeder in das Gehäuse eingeschoben wird. Wird die Kontaktkugel auf eine Fläche aufgesetzt, so wird die Kontaktkugel in Richtung auf das Gehäuse bewegt und der von dem Gehäuse verdeckte Marker freigegeben.

Aus der nach dem Anmeldetag dieser Anmeldung veröffentlichten WO 2004/082489 A1 ist ein Instrument zum Fixieren der Position einer Schneidebene bekannt, wobei an einer in einen Schneidblock einsteckbaren Platte mehrere aufgebogene flexible Blattelemente vorgesehen sind, um ein möglicherweise vorhandenes Spiel zu beseitigen

Es ist die Aufgabe der vorliegenden Erfindung eine Vorrichtung zum Bestimmen der Position eines Schneidblockes oder eines medizinischen Operationsinstrumentes vorzuschlagen, welche eine sichere Positionsbestimmung auch nach mehrmaliger Verwendung sicherstellen können.

Diese Aufgabe wird durch die Merkmale des Anspruchs 1 gelöst. Vorteilhafte Ausführungsformen ergeben sich aus den Unteransprüchen.

Die erfindungsgemäße Vorrichtung zum Bestimmen der Position eines Schneidblockes weist ein Positionierungselement, wie zum Beispiel einen Referenzstern mit mindestens einem Referenzpunkt oder Marker auf, dessen Position im Raum erfasst werden kann. Bei dem Positionierungselement kann es sich um aktive und/oder passive Elemente handeln, die geeignete Signale abgeben bzw. reflektieren, welche von entsprechenden Aufnahmegeräten, wie zum Beispiel Kameras, erfasst werden, um so die räumliche Lage des Positionierungselements und der damit verbundenen Vorrichtung bestimmen zu können. Weiterhin ist an der erfindungsgemäßen Vorrichtung ein Positionsbestimmungselement vorgesehen, welches fest mit dem Positionierungselement verbunden ist und welches mit einem Instrument oder einer Vorrichtung, wie zum Beispiel einem Schneidblock oder einer anderen Vorrichtung, welche eine zu navigierende Fläche aufweist, verbunden werden kann. Erfindungsgemäß besteht das Positionsbestimmungselement aus einem Referenzelement, wie zum Beispiel einer Referenzplatte, deren eine Oberfläche als Anlage an eine Fläche bei einem zu navigierenden Instrument dienen kann und als Referenzebene dient, wobei erfindungsgemäß mindestens ein Andruckelement oder Spannelement vorgesehen ist, welches es ermöglicht, dass die Anlagefläche des Referenzelementes gegen eine Fläche eines zu navigierenden Instrumentes gedrückt werden kann, wenn das Positionsbestimmungselement in eine Öffnung oder einen Schlitz eingesteckt wird. Die mindestens eine erfindungsgemäße Andruckplatte kann so ausgebildet sein, dass ein oder mehrere Andruckelemente oder Andruckplatten mit einer zum Beispiel durch eine Feder oder ein Spannelement erzeugten Kraft von dem Referenzelement weggedrückt werden, wodurch das Referenzelement in Verbindung mit dem mindestens einen vorgespannten Andruckelement in einer Öffnung oder einem Schlitz festsitzend verspannt oder eingeklemmt werden kann, unabhängig davon, ob die Öffnung oder der Schlitz eine genau vorgegebene Dicke aufweisen oder zum Beispiel geringfügig von dieser vorgegebenen Dicke abweichen. Das durch eine Kraft vorgespannte Andruckelement kann somit erfindungsgemäß verhindern, dass das Positionierungselement aufgrund eines Spiels in keinem genau definierten Lageverhältnis zu dem mit dem Positionierungselement verbundenen Instrument steht.

Vorzugsweise ist das Positionsbestimmungselement oder ein Teil davon, d. h. das Referenzelement und/oder das Andruckelement, um eine Achse relativ zum Positionierungselement oder Referenzstern drehbar, wobei die Achse vorzugsweise parallel zu einer Normalen auf der durch das Referenzelement bestimmten Referenzebene ist. Ebenso ist es vorteilhaft, wenn diese Elemente verschiebbar sind, wobei vorteilhaft das Referenzelement so ausgebildet ist, dass eine Anlagefläche an dem Referenzelement, welches eine Referenzebene bildet, nur innerhalb der Referenzebene verschoben werden kann.

Als Positionierungselement ist bevorzugt ein Referenzstern mit drei darauf angeordneten passiven Markern vorgesehen, welche zur Navigation der durch das Positionsbestimmungselement gebildeten Referenzebene verwendet werden können.

Vorteilhaft ist das Referenz- und/oder Andruckelement mindestens eine in einen Schneidblock einsteckbare Platte, wobei auch mehrere einzelne bevorzugt in einer Ebene liegende Plattenelemente als Referenz- und/oder Andruckelement verwendet werden können. Das Andruckelement ist bevorzugt in einer Richtung senkrecht zu der durch das Referenzelement gebildeten Anlage- oder Referenzebene bewegbar. Vorteilhaft kann ein Teil des Andruckelements so ausgebildet sein, dass es zum Beispiel in eine Aussparung des Referenzelements gebracht werken kann und zum Beispiel etwa die gleiche Dicke aufweist wie die Dicke einer als Referenzelement verwendeten Platte, so dass das Referenzelement zusammen mit dem Andruckelement in einen Schlitz eingesteckt werden kann.

Vorzugsweise wird das Andruckelement durch ein Feder- oder Vorspannelement mit einer Kraft beaufschlagt, welche aus der durch das Referenzelement definierten Ebene heraus wirkt und bevorzugt senkrecht auf dieser Ebene steht, so dass beim Einstecken des Referenzelements mit dem Andruckelement in einen Schlitz das Andruckelement aufgrund des Vorspannelements so weit von dem Referenzelement wegbewegt wird, bis das Andruckelement und das Referenzelement fest an Seitenflächen eines Schlitzes oder einer Öffnung anliegen und somit eine feste und definierte Verbindung zwischen dem Positionierungselement und dem Instrument herstellen können. Das Vorspannelement kann zum Beispiel durch eine oder mehrere Federn gebildet werden, wobei beispielsweise eine Spiralfeder zwischen dem Referenzelement und dem Andruckelement so vorgesehen ist, dass die Feder komprimiert werden muss, um das Referenzelement auf das Andruckelement oder in eine Aussparung des Andruckelements hinein zu bewegen, wodurch die Feder eine Rückstellkraft erzeugt, mit welcher das Referenzelement wieder vom Andruckelement weggedrückt wird, um durch das Zusammenwirken des Referenzelements mit dem Andruckelement zum Beispiel eine kraftschlüssige oder auch formschlüssige Verbindung mit einem Schlitz, welcher an einem Instrument vorgesehen ist, herstellen zu können. Das Federelement kann sowohl als Druckfeder, als auch als Zugfeder oder als Blattfeder ausgebildet sein und sollte an der erfindungsgemäßen Vorrichtung so vorgesehen sein, dass das Referenzelement von dem Andruckelement weg bewegt wird.

Vorteilhaft kann das Vorspannelement oder ein Federelement an der erfindungsgemäßen Vorrichtung so vorgesehen sein, dass durch die vom Vorspannelement erzeugte Kraft eine Drehbewegung eines frei drehbaren Positionierungselementes oder Referenzsternes gehemmt werden kann und bevorzugt das Positionierungselement oder der Referenzstern in einer festen Position festgestellt oder arretiert werden kann.

Gemäß einem weiteren Aspekt bezieht sich die Erfindung auf ein System zum Positionieren eines Schneidblockes mit einer wie oben beschriebenen Vorrichtung und einem Schneidblock, welcher mindestens einen Schlitz aufweist, welcher als Führungsschlitz verwendet werden kann und in welchen das Positionsbestimmungselement und insbesondere eine Platte eingesteckt werden kann, welche fest mit dem Positionierungselement oder Referenzstern verbunden ist.

Ebenso kann die beschriebene Vorrichtung auch zum Navigieren anderer Instrumente verwendet werden, welche eine Öffnung oder einen Schlitz zum Einstecken des Positionsbestimmungselementes aufweisen.

Demzufolge ermöglicht die vorliegende Erfindung das definierte und spielfreie Anbringen eines Positionierungselementes oder Referenzsternes an einem Schlitz oder einer Öffnung eines Instruments, wobei der Schlitz oder die Öffnung unterschiedliche Dicken aufweisen können, und wobei durch die Erfindung sichergestellt werden kann, dass eine durch ein Referenzelement gebildete Referenzebene navigiert werden kann, wenn der Abstand des Positionierungselements zum Referenzelement oder zu einer als Referenzebene dienenden Anlagefläche der Vorrichtung definiert ist und zum Beispiel einen konstanten festgelegten Abstand hat, wobei das zum Festklemmen verwendete Andruckelement eine sichere Befestigung an Öffnungen oder Schlitzen mit verschiedener Dicke ermöglicht, so dass die erfindungsgemäße Vorrichtung in einer Software als vorkalibrierte Vorrichtung implementiert werden kann. Es ist auch möglich, dass eine als Referenzebene dienende Anlagefläche zur Verifikation oder Überprüfung der Position einer Oberfläche, wie zum Beispiel eines Schnittes an einem Knochen, verwendet werden kann.

Die Erfindung wird nachfolgend anhand von Ausführungsbeispielen beschrieben werden.

Es zeigen:
- Figuren 1 und 2A-C: eine erste Ausführungsform einer erfindungsgemäßen Vorrichtung;
- Figuren 3 bis 5: eine zweite Ausführungsform der erfindungsgemäßen Vorrichtung;
- Figuren 6 bis 8: eine dritte Ausführungsform der erfindungsgemäßen Vorrichtung;
- Figuren 9 bis 11: eine Ausführungsform einer Vorrichtung zur Bestimmung der Position eines Schneidblocks;
- Figuren 12A bis 12B: verschiedene Ansichten eines tibialen Schneidblocks;
- Figur 13: zwei Ansichten eines femoralen Schneidblocks;
- Figuren 14A und 14B: das Schneiden eines Knochens mit einem positionierten Schneidblock;
- Figur 15: das Schneiden der Schnittebenen S1 bis S6 mit einem zweiten Schneidblock;
- Figur 16: eine bekannte Vorrichtung zur Positionierung eines Schneidblocks; und
- Figur 17: die Positionierung eines Schneidblockes nach dem Stand der Technik;

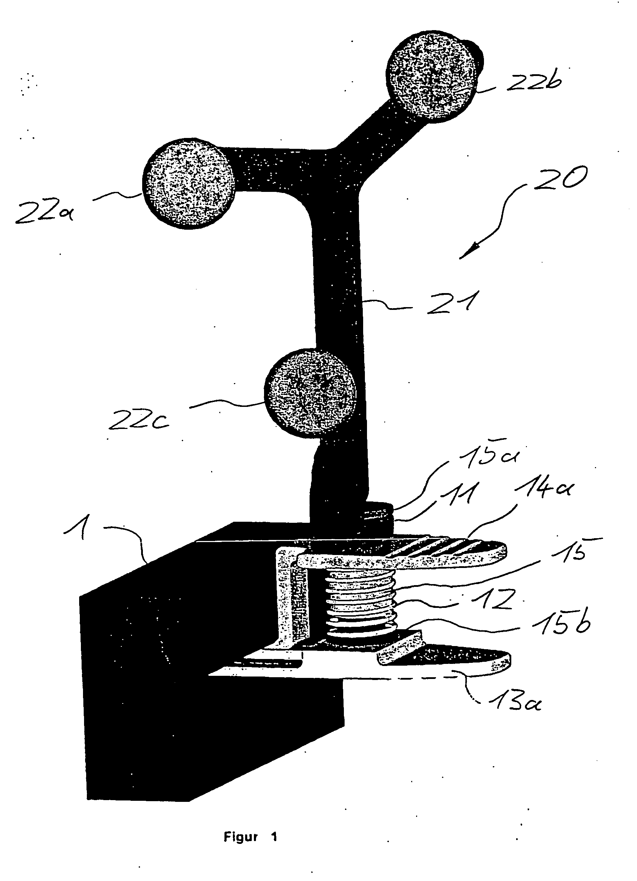

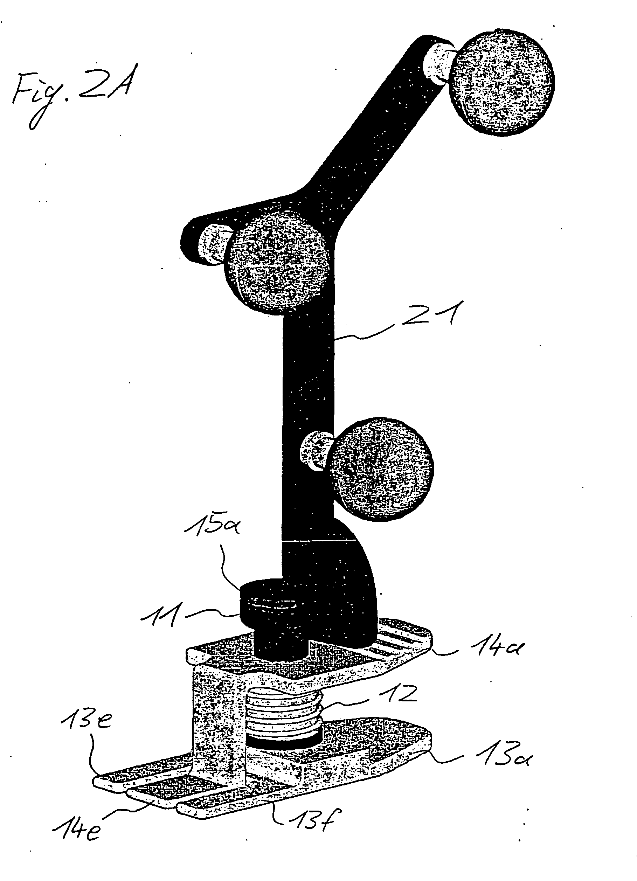

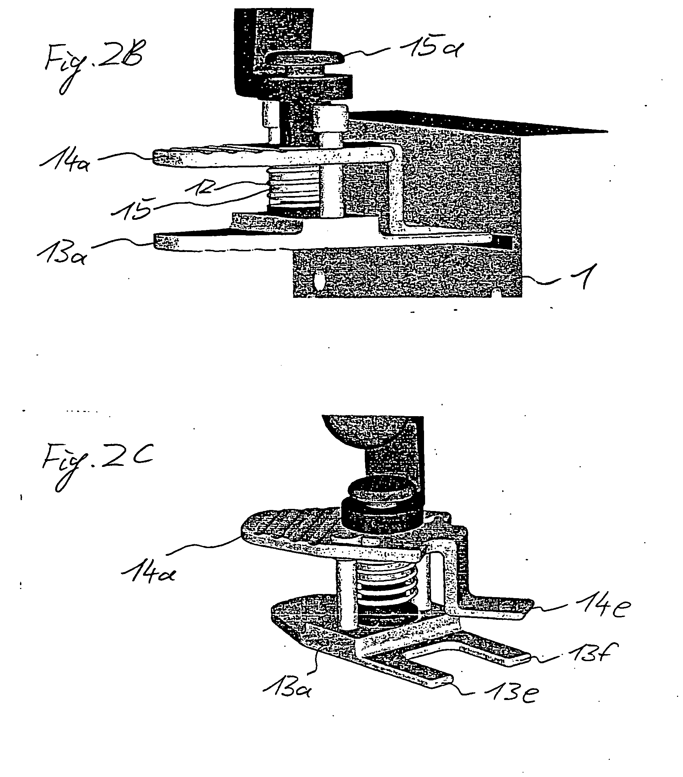

Die Figuren 1 und 2A bis C zeigen eine erfindungsgemäße Vorrichtung 20 zum Bestimmen der Position eines Schneidblockes 1 mit einem Referenzstern 21, an welchem drei als reflektierende Kugeloberflächen ausgebildete Marker 22a, 22b und 22c befestigt sind. Der Referenzstern 21 ist über ein eine Drehbewegung ermöglichendes Verbindungselement 11, welches als Klammer oder Buchse ausgebildet sein kann. Mittels des Verbindungselements 11 ist der Referenzstern 21 drehbar an dem als Achse ausgebildeten Referenzsternhalter 15 befestigt, welcher an seiner Ober- und Unterseite jeweils tellerförmige Abschlusselemente 15a und 15b aufweist. Das in Figur 1 gezeigte obere Abschlusselement 15a verhindert, dass sich das Verbindungselement 11 vom Referenzsternhalter 15 lösen kann. Der Referenzsternhalter 15 ragt durch eine Öffnung des Andruckelements 14a hindurch und ist auf der Unterseite mit dem unteren Abschlusselement 15b mit dem Referenzelement 13a verbunden, dessen Unterseite als Referenzebene dient. Auf der Oberseite des unteren Abschlusses 15b stützt sich eine Feder 12a ab, welche gegen die Unterseite des Andruckelements 14a drückt und somit das Andruckelement 14a von dem Referenzelement 13a weg drückt. Das Andruckelement 14a ist stufenförmig ausgebildet und weist eine Andruckplatte 14e auf, welche zwischen zwei Referenzplatten 13e und 13f des Referenzelementes 13a geschoben werden kann wie in Figur 2 gezeigt.

Ein Benutzer kann zum Beispiel das Andruckelement 14a und das Referenzelement 13a zwischen zwei Finger nehmen und durch ein Zusammendrücken der Elemente 13a und 14a die zwischen diesen Elementen vorgesehener Feder 12a spannen und die Vorrichtung in einen Schlitz eines Schneidblockes 1 einschieben, so dass nach dem Loslassen der Vorrichtung 20 das Andruckelement 14a durch die Kraft der Feder 12a relativ zum Referenzelement 13a nach oben verschoben wird, um die Vorrichtung fest mit dem Schneidblock 1 zu verbinden, wobei das Lageverhältnis zwischen dem Referenzstern 21 und der durch die Unterseite des Referenzelementes 13a gebildete Referenzebene konstant bleibt, so dass die an die Referenzebene anliegende Oberfläche des Schlitzes im Schneidblock 1 mittels des Referenzsternes 21 navigiert werden kann. Aufgrund der auf das untere Abschlusselement 14b wirkende Federkraft wird der Referenzstern 21 in dem in Figur 1 gezeigten Zustand der erfindungsgemäßen Vorrichtung arretiert und kann nicht mehr gedreht werden.

Figur 2C zeigt die in den Figuren 1, 2A und 2B gezeigte Vorrichtung, wobei das Andruckelement 14a zur Ermöglichung einer einfachen Reinigung der Vorrichtung von dem Referenzelement 13a weg bewegt worden ist.

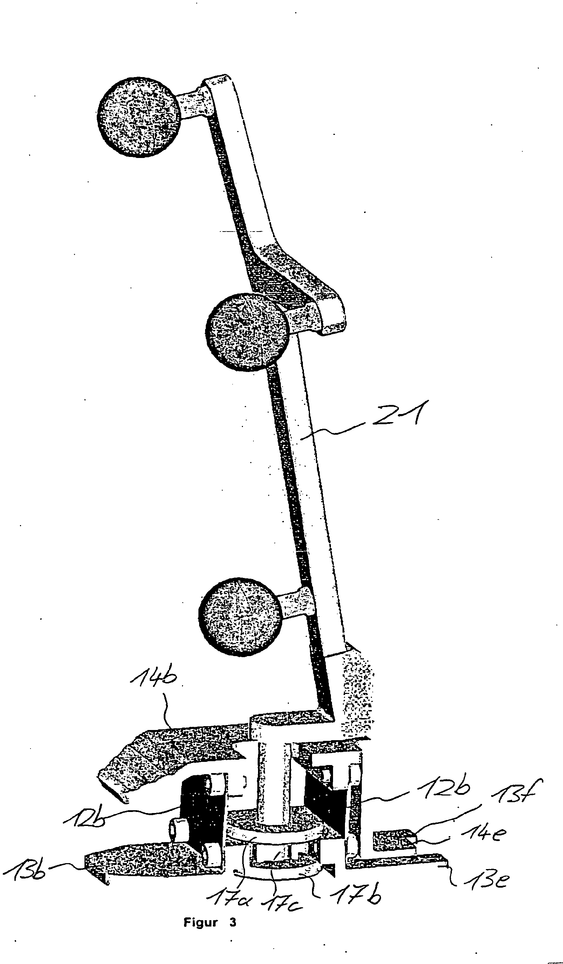

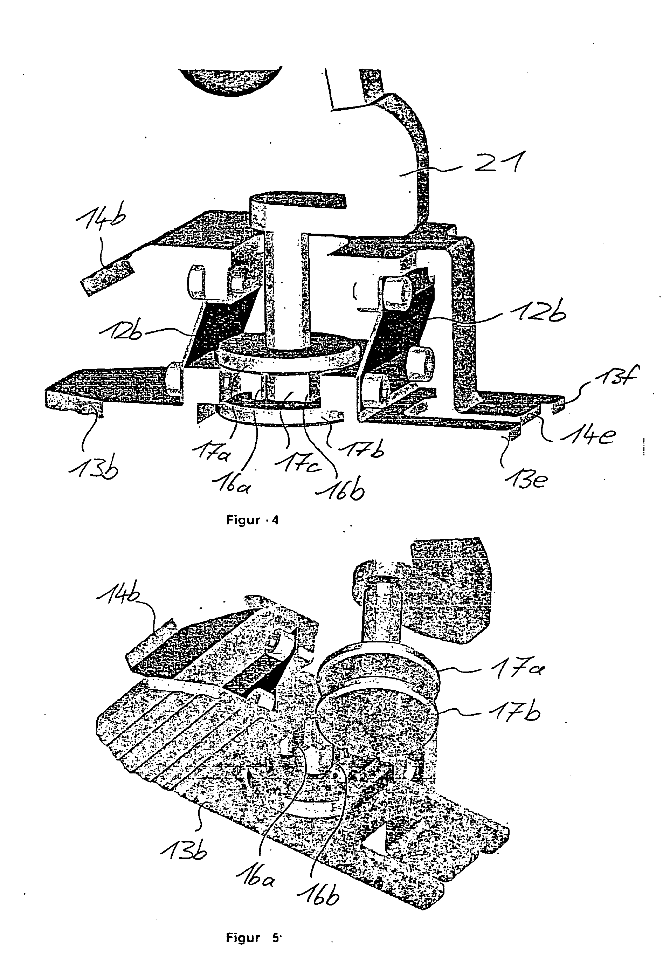

Die Figuren 3 bis 5 zeigen eine zweite Ausführungsform der erfindungsgemäßen Vorrichtung mit einem Referenzstern 21, welcher zwei Ringe 17a und 17b aufweist, die durch ein Abstandsstück 17c voneinander getrennt sind. Das Abstandsstück 17c des Referenzsternes 21 kann zwischen zwei eine Klemmstelle bildende Rastelementen 16a und 16b mit dem Referenzelement 13b verbunden werden. Das Referenzelement 13b ist über zwei etwa vertikal zum Referenzelement 13b verlaufende Blattfedern 12b mit einem Andruckelement 14b verbunden, wobei wie in Figur 4 gezeigt das Andruckelement 14b durch eine seitliche Verschiebung der Blattfedern 12b aus der in Figur 3 gezeigten Ausgangsstellung so bewegt werden kann, dass die Andruckplatte 14e zwischen die Referenzplatten 13e und 13f des Referenzelementes 13b bewegt wird. Wenn die in Figur 4 gezeigten Referenzplatten 13e und 13f zusammen mit der Andruckplatte 14e in einen Schlitz oder eine Öffnung eines Instrumentes eingefügt werden, so erzeugen die Blattfedern 12b ein Rückstellkraft des Andruckelements 14b relativ zu dem Referenzelement 13b, wobei auch eine Komponente der Rückstellkraft senkrecht zur unteren Oberfläche des Referenzelementes 13b wirkt, welche als Referenzebene dient, wodurch die in den Figuren 3 bis 5 gezeigte Vorrichtung durch die Bewegung der Andruckplatte 14e relativ zu den Referenzplatten 13e und 13f spielfrei an einer Öffnung oder in einem Schlitz befestigt werden kann.

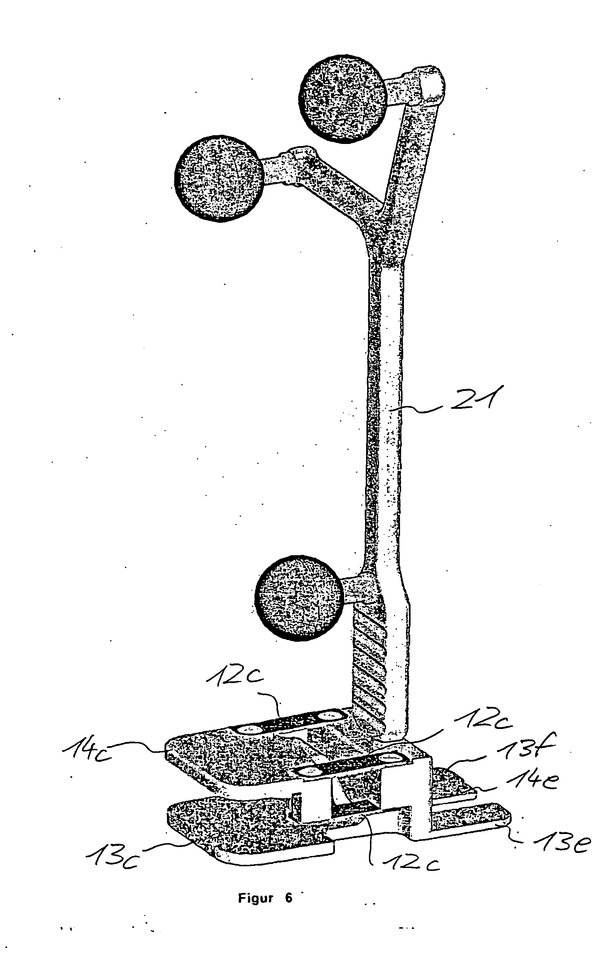

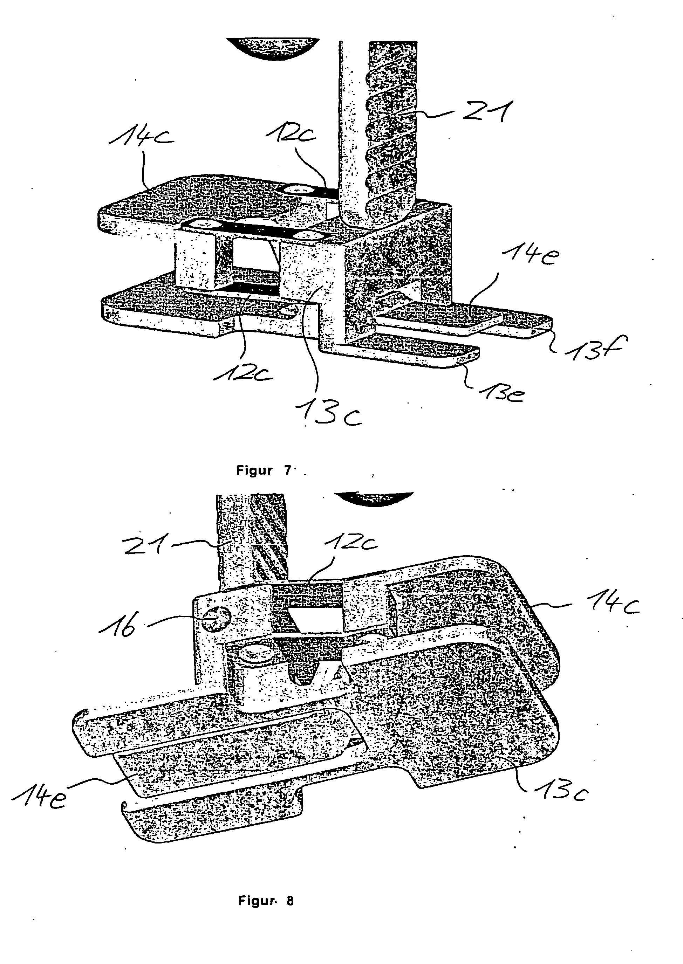

Eine dritte Ausführungsform der erfindungsgemäßen Vorrichtung ist in den Figuren 6 bis 8 gezeigt, wobei das Andruckelement 14c über etwa horizontal bzw. parallel zur Unterseite des Referenzelementes 13c verlaufende Blattfedern 12c mit dem Referenzelement 13c verbunden ist, wobei wiederum durch die Blattfedern 12c eine auf die Andruckplatte 14e wirkende Kraft erzeugt werden kann, so dass sich die Andruckplatte 14e aus der durch die Referenzplatten 13e und 13f des Referenzelementes 13c gebildeten Ebene heraus bewegt werden kann, um den Referenzstern 21 fest und in einem definierten Lageverhältnis relativ zur Unterseite des Referenzelementes 13a mit einer Öffnung oder einem Spalt zu verbinden.

Der Referenzstern 21 kann mittels eines in das Referenzelement 13c einschiebbaren Kugeldruckstiftes 16 mit dem Referenzelement 13c verrastet werden.

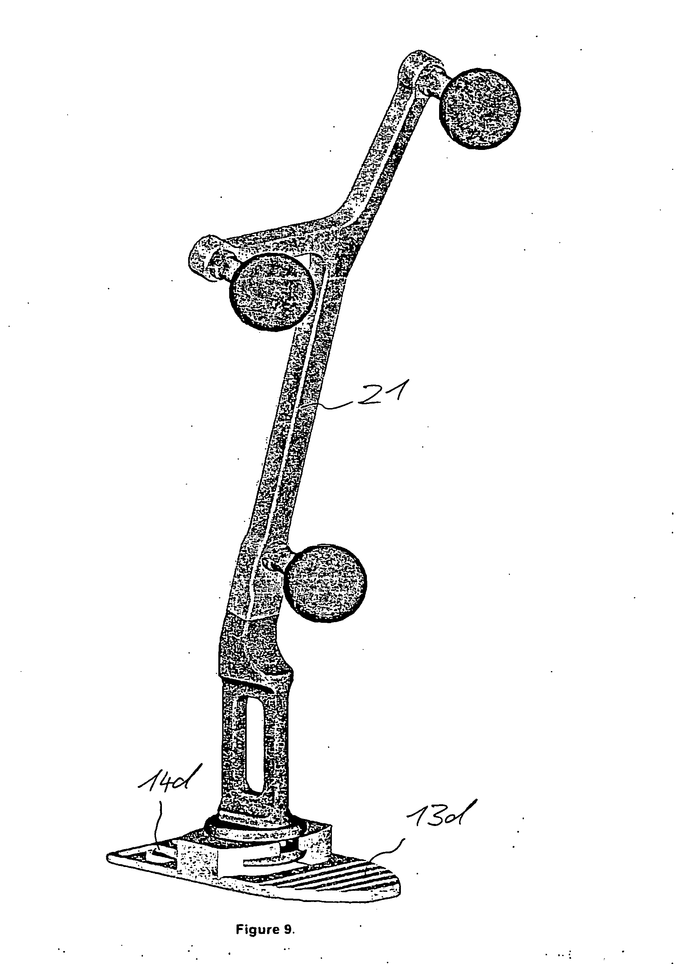

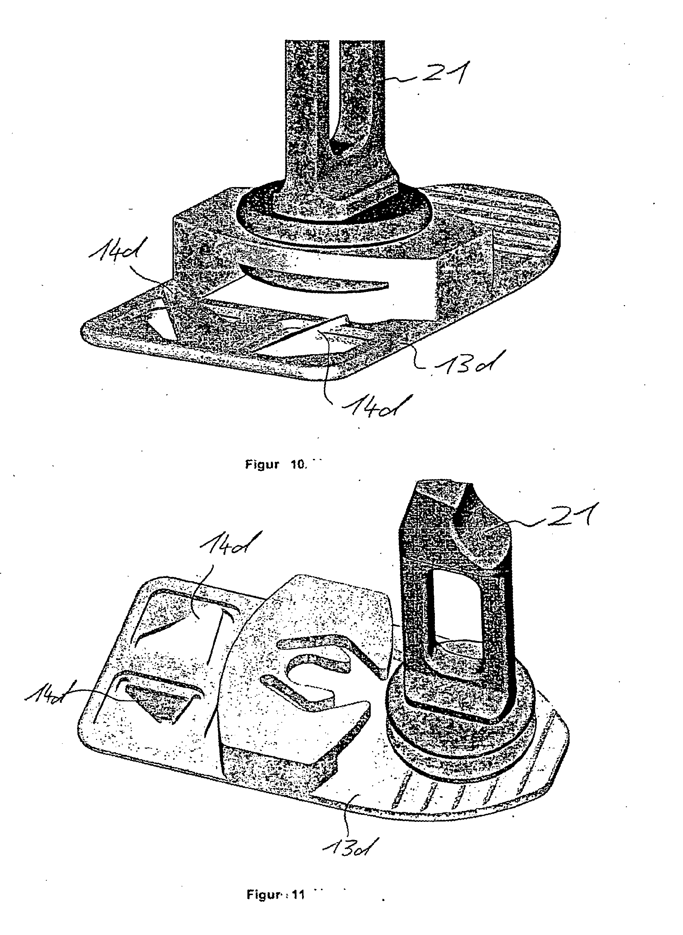

Die Figuren 9 bis 11 zeigen eine Ausführungsform einer Vorrichtung zur Bestimmung der Position eines Schneidblocks, wobei der Referenzstern 21 mit dem Referenzelement 13d auf ähnliche Weise wie im zweiten Ausführungsbeispiel gezeigt verrastet werden kann. Anders als bei den vorhergehenden Ausführungsbeispielen ist kein durch eine separate Feder vorgespanntes Andruckelement vorgesehen, da die zur spielfreien Positionierung der Unterseite des Referenzelementes 13d in einer Öffnung oder einem Spalt erforderliche Andruckkraft durch die aus dem Referenzelement 13d herausragenden elastischen oder Federelemente 14d erzeugt wird, welche aufgrund ihrer in Einschubrichtung vorhandenen Abschrägung das Einschieben des Referenzelementes 13d in einen Spalt dadurch ermöglichen, dass während des Einschiebens die elastischen oder Federelemente 12d in die Ebene des Referenzelementes 13d hineingedrückt werden und im eingeschobenen Zustand gegen eine der Unterseite des Referenzelementes 13d gegenüberliegende Fläche einer Öffnung oder eines Spaltes drücken. Diese Ausführungsform ist nicht Teil der Erfindung.

## Patentansprüche

1. Vorrichtung (20) zum Bestimmen der Position eines Schneidblockes (1) mit einem Positionierungselement (21) mit mindestens einem Referenzpunkt (22a, 22b, 22c), dessen räumliche Position erfasst werden kann, mit einem elastischen oder Federelement (12) und mit einem Positionsbestimmungselement (13, 14), wobei das Positionsbestimmungselement mindestens ein eine Anlageebene definierendes Referenzelement (13), welches mindestens eine in einen Schlitz eines Schneidblocks (1) einsteckbare Platte ist, aufweist, wobei das Positionsbestimmungselement mindestens ein Andruckelement (14) aufweist und wobei das Andruckelement eine in den Schlitz des Schneidblocks (1) einsteckbare Andruckplatte (14e) aufweist, die durch das elastische oder Federelement (12), das zwischen dem Referenzelement (13) und dem mindestens einen Andruckelement (14) vorgesehen ist, mit einer Kraft beaufschlagt wird.

2. Vorrichtung nach Anspruch 1, wobei die Andruckplatte (14e) des Andruckelementes (14) in eine Plattenebene des Referenzelementes (13) bewegt werden kann.

3. Vorrichtung nach einem der vorhergehenden Ansprüche, wobei das Positionierungselement (21) durch das elastische Element oder Federelement (12) festgestellt oder arretiert werden kann.

4. Vorrichtung nach einem der vorhergehenden Ansprüche, wobei das Positionierungselement ein Referenzstern (21) ist und bevorzugt mindestens drei Elemente (22a, 22b, 22c) mit reflektierender Oberfläche als Referenzpunkte aufweist.

5. System zum Positionieren eines Schneidblockes (1) mit einer Vorrichtung (20) nach einem der vorhergehenden Ansprüche und einem Schneidblock (1).

## Claims

1. Device (20) for determining the position of a cutting bock (1), with a positioning element (21) and at least one reference point (22a, 22b, 22c), the spatial position of which can be detected, with an elastic or spring element (12) and with a position-determining element (13, 14), which position-determining element has at least one reference element (13) defining a contact plane comprising at least one plate which can be inserted in a slot of a cutting block (1), and the position-determining element has at least one pressing element (14) and the pressing element has a pressing plate (14e) which can be inserted in the slot of the cutting block (1) and a force is applied to it by the elastic or spring element (12) disposed between the reference element (13) and the at least one pressing element (14).

2. Device as claimed in claim 1, wherein the pressing plate (14e) of the pressing element (14) can be moved into a plate plane of the reference element (13).

3. Device as claimed in one of the preceding claims, wherein the positioning element (21) can be fixed or locked by means of the elastic element or spring element (12).

4. Device as claimed in one of the preceding claims, wherein the positioning element is a reference star (21) and preferably has at least three elements (22a, 22b, 22c) with a reflecting surface as reference points.

5. System for positioning a cutting guide (1) with a device (20) as claimed in one of the preceding claims and a cutting block (1).

## Revendications

1. Dispositif (20) pour déterminer la position d'un guide de coupe (1) avec un élément de positionnement (21) avec au moins un point de référence (22a, 22b, 22c) dont la position spatiale peut être relevée, avec un élément élastique ou élément de ressort (12) et avec un élément de détermination de position (13,14), dans lequel l'élément de détermination de position comporte au moins un élément de référence (13) qui définit un plan d'application et qui est au moins une plaque à insérer dans une fente d'un guide de coupe (1), dans lequel l'élément de détermination de position comporte au moins un élément de pression et dans lequel l'élément de pression comporte une plaque de pression (14e) qui peut être insérée dans la fente du guide de coupe et qui est sollicitée par l'élément élastique ou élément de ressort (12) qui est prévu entre l'élément de référence (13) et le au moins un élément de pression (14).

2. Dispositif selon la revendication 1 dans lequel la plaque de pression (14e) de l'élément de pression (14) peut être déplacée dans un plan de plaque de l'élément de référence (13).

3. Dispositif selon l'une quelconque des revendications 1 ou 2 dans lequel l'élément de positionnement (21) peut être fixé ou bloqué par l'élément élastique ou élément de ressort (12).

4. Dispositif selon l'une quelconque des revendications 1 à 3 dans lequel l'élément de positionnement (21) est une étoile de référence (21) et comporte de préférence au moins trois éléments (22a, 22b, 22c) avec surface réfléchissante servant de points de référence.

5. Système pour positionner un guide de coupe (1) avec un dispositif (20) selon l'une quelconque des revendications 1 à 4 et avec un guide de coupe (1).
